# EUROPEAN PATENT SPECIFICATION

(11) **EP 1 929 979 B1**
(45) Date of publication and mention of the grant of the patent: **31.08.2011**
(21) Application number: 07254545.2
(22) Date of filing: 22.11.2007
(51) Int. Cl.: A61F 2/84

(54) **Spring stop for stent delivery system and delivery system provided with same**
Federanschlag für ein Stenteinführungssystem und damit ausgestattetes Einführungssystem
Butée amovible pour système de pose d'endoprothèse vasculaire et système de pose fourni avec celui-ci

(30) Priority: 07.12.2006 US 567785
(43) Date of publication of application: 11.06.2008
(73) Proprietor: Cordis Corporation, Miami Lakes, FL 33014 (US)
(72) Inventor: Boucher, Donald D., Boynton Beach, FL 33437 (US); Buzzard, Jon D., Miramar, FL 33029 (US)
(74) Representative: Belcher, Simon James

(56) References cited:
- EP-A- 1 803 423
- US-A- 5 707 376
- US-A1- 2002 151 955
- US-A1- 2005 228 478

## Description

The present invention generally relates to medical devices, particularly to a SDS (stent delivery system) that incorporates a spring stent stop. The delivery system, owing to a novel stent stop construction, can be assembled, through the crimped stent in place within the outer sheath of the device, enabling the device to be assembled with considerably greater efficiency by eliminating process steps and component(s).

A stent is a generally longitudinal tubular device formed of biocompatible material(s) that is useful in the treatment of stenoses, strictures or aneurysms in blood vessels and other body vessels. Stents can be implanted within an unhealthy vessel to reinforce collapsing, partially occluded, weakened, or abnormally dilated sections of the vessel. Typically, stents are employed after angioplasty of a blood vessel to maintain vessel patency of the diseased lesion of the vessel. While stents are most notably used in blood vessels, stents may also be implanted in other body vessels such as the urogenital tract and the bile duct. A stent may exhibit flexibility to allow it to be inserted through curved vessels. Furthermore, Nitinol (self expanding) stents are often compressed radially, such as by crimping, to facilitate placement into the delivery system in preparation for deployment using intraluminal catheter implantation.

Stents are akin to scaffoldings in their support of the passageway. Structurally, a stent may have two or more struts or wire support members connected together into a lattice-like frame. As indicated, stents may be in a compressed state prior to delivery and deployment, as compression facilitates insertion through small cavities. Stents can be delivered to the desired implantation site percutaneously in a catheter or similar transluminal device. With a lattice-like structure, a portion of the stent surface area is open, such openings defined by the struts that form the stent.
Typically, during delivery of self-expanding stents, the delivery device encloses the stent within the distal sheath. The stent and sheath can be advanced to a site within the patient's vessel through a guide catheter. A self-expanding stent possesses chronic outward force (COF) when warmed above the martensitic transition temperature for the nitinol alloy (e.g., above 30° C), which causes the stent to expand to a pre-determined diameter following its deployment in the vessel when the encasing sheath is retracted from the compressed stent.

Stent delivery systems (SDS) for self-expanding stents are generally comprised of an inner shaft or shafts terminating distally in a stop, from which a separate wire lumen extends further distally, and at the end of such wire lumen is provided a distal tip. The inner shaft of stent delivery systems disclosed in the art may have a proximal portion made from a flexible coiled member, akin to a compressed or closed coil spring, and a distal wire lumen that may be polymeric in constitution, such as a construct formed of a coextrusion of high density polyethylene and nylon. The stent resides within the distal outer sheath of the device. The outer sheath is comprised of various segments joined together by any number of means known to those of ordinary skill in the art including heat fusing, adhesive bonding, chemical bonding or mechanical attachment. The prior art further shows that a stop is fixed to the inner shaft in the vicinity of where the flexible coiled member and distal shaft portion converge. The diameter of the stop should provide sufficient surface area to maintain relative position of the stent during deployment, that is, the stop should provide a counterforce against stent movement as the outer shaft is pulled in the proximal direction as part of the stent deployment process.

The stent is crimped and loaded into an outer sheath. The stent has to be loaded into the outer sheath, a process that renders the construction and assembly of the delivery device highly complex. Furthermore typical construction of the distal region of a delivery device is complicated because the outer sheath is typically a component separate from the inner shaft member. Thus, the inner shaft and the outer sheath must be assembled in a specific order in order to have the stop proximal of the stent.

US-5707376 relates to a stent introducer. One embodiment includes an inner member with a distal end protuberance, comprising a forked cannula. The protuberance engages the distal end of a stent to pull the collapsed stent from an outer member passage. The protuberance is used with a ratcheting motion in and out of the outer member passage.

US-A-2002/0151955 relates to a stent delivery system. It includes a shape memory metal bumper adjacent the proximal end of the stent. The bumper can be expanded from a reduced profile state to an increased profile state by increasing the temperature of the bumper.

According to an aspect of the present invention, there is provided a delivery device for delivering and deploying a medical implant as defined in appended claim 1.

According to another aspect of the present invention, there is provided a method for preparing a medical implant delivery device as defined in appended claim 2.

The single spring stent stop used in the present invention exhibits spring-like characteristics. The stop is compressible preferably in a radially inward direction when a sufficient compressive force is applied, yet expands to its original non-compressed state when such force is removed. When in the desired position on the axially extending member of the medical implant delivery device, the stop provides a rigid surface positioned to contact the proximal stent end. That is, when the stent is positioned upon the axially extending member, such as the inner shaft of the medical implant delivery device, the rigid surface of the stop contacts the stent, maintaining the position of the stent during deployment. This function is advantageous as the outer sheath is retracted proximally during deployment, an action that but for the presence of the stop, could cause the stent to be deployed outside the target site. This configuration allows the inner sheath assembly (with stop affixed) to be assembled by passing the proximal end of the inner sheath assembly through the distal outer member and have the loaded stent already in place. The inner sheath could eliminate the need for a separate guidewire sheath, as the tip of the device can be over-molded directly on the inner sheath prior to assembly.

The spring stent stop can be constructed of elements arrayed to define a through hole sized to receive the inner shaft of the medical implant delivery device.

The spring stent stop may be provided with elements having a first base end from which a plurality of fingers distally project. The fingers are radially compressible inward, in a direction extending towards the inner shaft member, yet biased to project radial openness at the distal end of the spring stent stop, which radial openness at the distal end is relatively greater than the radial openness of the base end (proximal end) of the device.

The spring stent stop may be sized and dimensioned so that when compressed, the stop has sufficiently reduced outer diameter to allow it to pass through a crimped stent as it moves proximally through the stent, and then into the desired position proximal to the stent. The particular aspect simplifies the preparation of the delivery device that employs the present spring stent stop in that the stop can be installed on the device after installation of the stent.

The spring stent stop may possess memory, so that upon compression, the stop returns to its original uncompressed dimensions once a compressive force is no longer applied to it. In the uncompressed state, the diameter of the distal end of the stop as manifested in the degree of openness of the fingers of the stop is substantially the same as the inner diameter of the outer member. Such memory can be provided by a biasing component, or material property of the material used in constructing the stop.

The spring stent stop may be affixed to the target location of residence upon the axially extending member by, for example, crimping, welding, or bonding it to the inner member. The stop member, ordinarily constructed of a relatively rigid material, can provide the inner member with an additional degree of column strength, preventing the inner sheath from collapsing and facilitating deployment of the stent while maintaining stent position at the target lesion.

In a specific embodiment, the stent delivery system requires no separate wire lumen distal to the stop, and further, construction of the inventive delivery system is simplified, as the distal tip is affixed to the inner member assembly subsequent to stent crimping and loading. That is, after the stent is loaded into the outer sheath, the inner member assembly, with the spring stent stop and tip previously affixed, can be moved into position so that the stent stop engages the proximal edge of the stent and the tip engages the distal outer sheath.

The delivery device of the present invention may be assembled by loading the device (e.g., a stent), crimping the stent, loading crimped stent into the outer member, passing a spring stent stop, such as described above, proximally through the stent, positioning the spring stent stop, aligning the distal tip to the outer sheath of the device, and then fixing the aligned inner member to proximal handle.

Embodiments of the invention will now be described by way of example with reference to the accompanying drawings, in which:
Figure 1 is an elevational view of a stent delivery system in that can be modified in accordance with the present invention;
Fig. 2 is a perspective view of a spring stent stop of the present invention passing through a stent; and
Fig. 3 is a second perspective view of a spring stent stop of the present invention positioned for delivery and deployment of the stent.

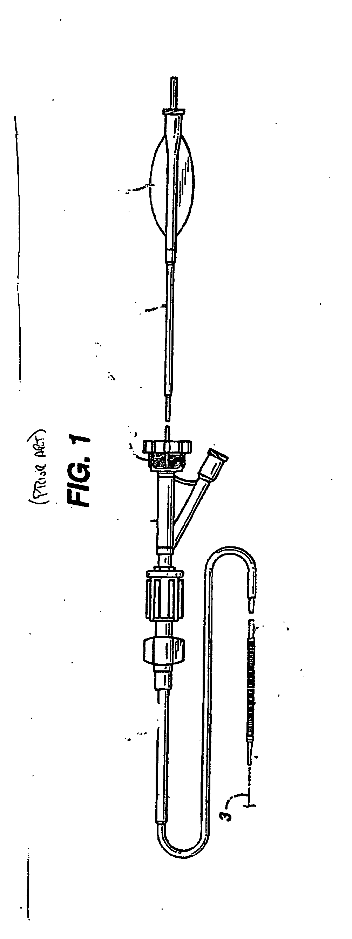

The structural attributes of the present invention may be incorporated into existing stent delivery systems. For that reason, the features of the stent delivery systems disclosed in US-6,773,446 and US-6,939,352, each of which share the assignee with the present application, are disclosed as an examples of stent delivery systems that can be modified to include the attributes within the ambit of this disclosure. However, the present invention need not be bound to the specific features and embodiments of these particular patent disclosures. In any event, Figure 1 illustrates a conventional stent delivery system of the kind disclosed in US-6,773,446.

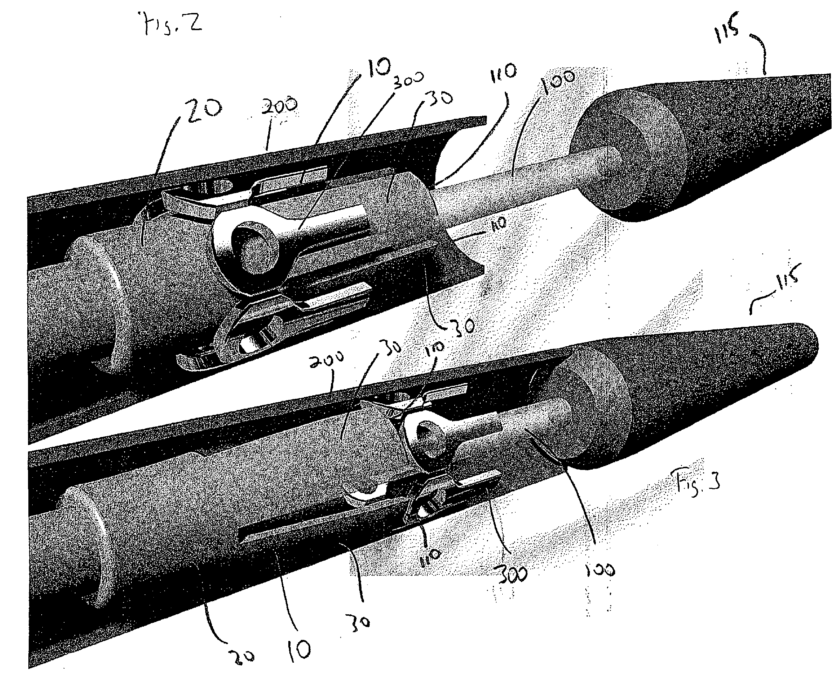

Figure 2 presents a perspective view of the present invention, in which spring stent stop 10 is shown at a position along the inner shaft member 100 of a stent delivery device. Specifically, the spring stent stop 10 is depicted at a position on the inner shaft member 100 that is proximal to distal tip 115 of delivery system. Here, the distal tip is shown in Figs. 1 and 2, though upon consideration of the entirety of the disclosure, it shall be evident that the affixing of the distal tip 115 to the inner shaft member 100 can take place prior to assembly or affixed after the spring stent stop is positioned proximal of the stent.

The spring stent stop 10 is constructed of finger elements 30, wherein the whole or part of such elements can be seen in Figures 2 and 3. The finger elements 30 extend distally from base portion 20.

Stop 10 can be made from any number of materials known in the art, including metals such as nitinol, stainless steel, or highly radio-opaque material such as platinum, gold, tantalum or polymers, including radio-opaque filled polymers. Providing a radio-opaque stop is advantageous and can aide in positioning the stent within the target lesion during deployment within a vessel.

The stop of the present invention is compressible in a radially inward direction when a sufficient compressive force is applied, and will expand to its original non-compressed state when such force is removed. The stop provides a rigid surface 110 located at the distal end of the stop that is positioned to contact the proximal stent end when the stop is deployed on the inner shaft. In other words, when the stent is positioned upon the inner shaft of the stent delivery system during deployment of the stent, the rigid surface of the stop contacts the stent, maintaining the position of the stent during deployment. This function is advantageous as the outer sheath 200 is retracted (i.e., moved proximally) during deployment, an action that but for the presence of the stop, could urge the stent to also move proximally.

In assembling the device, the spring stent stop and distal tip, which previously are affixed to the inner member, is loaded through the crimped stent within the outer member and positioned proximal of the stent.

Upon passing through the crimped stent, the spring stop takes its position proximal to the crimped stent. Thus the crimped stent, and outer sheath portion of the shaft, reside between the spring stop and the distal tip.

After the spring stop 10 passes through the crimped stent, the spring like features of the stent, and biasing action associated therewith, cause the fingers 30 to open to a diameter that is substantially the same as the inside diameter of the outer member 200.

The spring like action of the stop creates a frictional engagement to shaft 10. When the distal fingers of the spring stop expand after passing through the crimped stent, the diameter of stop 10 is large enough to make sufficient contact with the proximal end of stent 50. The frictional contact with the outer sheath 200 does not adversely affect stent deployment. As explained above, stop 10 helps to maintain the relative position during deployment, by preventing the stent from being deployed proximal of target lesion. The radio-opaque stop 10 also aides in positioning the stent within the target lesion during deployment within a vessel, as is described below.

One of the advantages provided by the present invention is that the separate distal component in the form of a wire lumen can be eliminated. That is, the delivery device can instead be constructed of only one inner shaft, thereby eliminating a separate wire lumen assembly as the tip can be affixed onto the inner member distal sheath, and therefore, the inner shaft can extend further than in heretofore known arrangements. Thus, the distal tip can be overmolded to the inner shaft with the spring stent stop already in place, which is yet another advantage offered by the present inventive stop construction.

To illustrate the advantages of the inventions disclosed herein, consider that with the present spring stop arrangement, the stent is crimped and loaded into the outer sheath. The inner shaft assembly is then slid through the crimped stent and outer sheath and positioned with the spring stent stop proximal of the crimped stent and the distal tip engaging the distal end of the outer sheath. The spring stent stop is moved to its location proximal to the crimped stent by passing the spring stent stop through the crimped stent, possibly by riding a tube, as explained above. The stent fingers, exhibiting spring-like movement in the radially inward direction (yet biased radially outward), are thus moved radially inward and retained in that position as the stop passes through the crimped stent. After the entire spring stop passes through the crimped stent, the fingers spring radially outward to their uncompressed position, with the fingers extending into a position that substantially abuts the proximal end of the crimped stent. Completing the delivery system construction, the distal tip is positioned to engage the distal outer member and the proximal inner member is joined (for example, by overmolding or adhesive bonding) to the handle to maintain alignment of the spring stent and distal tip relative to the stent.

It will be understood that this disclosure, in many respects, is only illustrative. Changes may be made in details, particularly in matters of shape, size, material, and arrangement of parts without exceeding the scope of the invention, as defined in the language of the appended claims.

## Claims

1. A delivery device for delivering and deploying a medical device to a location in a patient's body requiring medical treatment, such as a blood vessel or duct, the delivery device comprising:
an outer sheath (200)
an operable portion comprising controls for moving the outer sheath (200);
a stent retained on an inner shaft and located within the outer sheath (200), which inner shaft is coaxial with the outer sheath (200);
a single stent stop (10) positioned proximal to the stent and comprising a body portion (20) and distally extending projections (30), wherein the body portion (20) and distally extending projections (30) are linked in an arrangement that defines an open passageway through an interior space in the stop (10); and wherein the single stent stop (10) is sized and single dimensioned to pass over the inner shaft and through the stent, and wherein the single stent stop (10) is adapted to affix to the inner shaft when the stop (10) is passes through the stent and assumes position proximal to the stent, and the distal projections (30) are in a substantially abutting arrangement with the stent when the single stop member (10) has assumed position proximal to the stent,
**characterised in that** the single stent stop is a spring stent stop and wherein the distally extending projections (30) are biased to an open position absent application of a sufficiently compressive force and upon application of a sufficiently compressive force upon the distally extending projections (30), the projections (30) are compressed towards the interior space.

2. A method for preparing a medical implant delivery device according to claim 1 for deployment comprised of the steps of:
positioning a medical implant on an axially extending member of the device;
passing the single spring stent stop through the stent to a position proximal of the stent until the stent frictionally engages the axially extending member; and
securing a distal tip component to the distal most end of the axial extending member.

## Patentansprüche

1. Einführungsvorrichtung zum Einführen einer medizinischen Vorrichtung an einen Ort in dem Körper eines Patienten, welcher einer medizinischen Behandlung bedarf, wie z.B. ein Blutgefäß oder einen Duktus, und zum dortigen Einsetzen, wobei die Einführungsvorrichtung Folgendes umfasst:
eine äußere Hülle (200),
einen betriebsfähigen Bereich, welcher Steuerungen zum Bewegen der äußeren Hülle (200) umfasst;
einen Stent, welcher auf einem inneren Schaft zurückgehalten ist und sich in der äußeren Hülle (200) befindet, wobei dieser innere Schaft koaxial zu der äußeren Hülle (200) ist;
einen Einzelstentanschlag (10), welcher proximal zu dem Stent positioniert ist und
einen Körperbereich (20) und sich distal erstreckende Vorsprünge (30) umfasst, wobei der Körperbereich (20) und die sich distal erstreckenden Vorsprünge (30) in einer Anordnung verbunden sind, welche einen offenen Durchgang durch einen Innenraum in dem Anschlag (10) definiert;
und wobei der Einzelstentanschlag (10) bemessen und dimensioniert ist, um über den inneren Schaft und durch den Stent zu gehen, und wobei der Einzelstentanschlag (10) eingerichtet ist, um an dem inneren Schaft angebracht zu sein, wenn der Anschlag (10) durch den Stent gegangen ist und eine Position proximal zu dem Stent eingenommen hat, und wobei die distalen Vorsprünge (30) in einer im Wesentlichen aneinandergrenzenden Anordnung mit dem Stent sind, wenn das Einzelanschlagselement (10) eine Position proximal zü dem Stent angenommen hat,
**dadurch gekennzeichnet, dass** der Einzelstentanschlag ein Federstentanschlag ist, und wobei die sich distal erstreckenden Vorsprünge (30) ohne Anwendung einer hinreichenden Druckkraft in eine offene Position vorgespannt sind, und wobei die Vorsprünge (30) bei Anwendung einer hinreichenden Druckkraft auf die sich distal erstreckenden Vorsprünge (30) in Richtung des Innenraums gepresst sind.

2. Verfahren zum Vorbereiten einer Einführungsvorrichtung für ein medizinisches Implantat gemäß Anspruch 1 für eine Einsetzung, wobei das Verfahren die folgenden Schritte umfasst:
Positionieren eines medizinischen Implantats auf einem sich axial erstreckenden Element der Vorrichtung;
Durchgehen des Einzelfederstentanschlags durch den Stent zu einer proximal zu dem Stent befindlichen Position, bis der Stent das sich axial erstreckende Element reibschlüssig einkoppelt; und
Befestigen einer Distalspitzenkomponente an dem distalsten Ende des sich axial erstreckenden Elements.

## Revendications

1. Dispositif de pose pour poser et déployer un dispositif médical à un emplacement dans le corps d'un patient nécessitant un traitement médical, tel qu'un vaisseau sanguin ou un conduit, le dispositif de pose comprenant :
une gaine externe (200) ;
une partie pouvant être actionnée comprenant des commandes pour déplacer la gaine externe (200) ;
un stent retenu sur une tige interne et positionné à l'intérieur de la gaine externe (200), dont la tige interne est coaxiale par rapport à la gaine externe (200) ;
une seule butée de stent (10) positionnée de manière proximale par rapport au stent et comprenant une partie de corps (20) et des saillies (30) s'étendant de manière distale, dans lequel la partie de corps (20) et les saillies (30) s'étendant de manière distale sont reliées dans un agencement qui définit une voie de passage ouverte à travers un espace intérieur de la butée (10) ; et dans lequel la seule butée de stent (10) est taillée et dimensionnée pour passer sur la tige interne et à travers le stent, et dans lequel la seule butée de stent (10) est adaptée pour se fixer sur la tige interne lorsque la butée (10) passe à travers le stent et prend une position proximale par rapport au stent, et les saillies distales (30) sont dans un agencement sensiblement en butée avec le stent lorsque le seul élément de butée (10) a pris la position proximale par rapport au stent ;
**caractérisé en ce que** la seule butée de stent est une butée de stent à ressort et dans lequel les saillies (30) s'étendant de manière distale sont sollicitées dans une position ouverte sans application d'une force suffisamment compressive et suite à l'application d'une force suffisamment compressive sur les saillies (30) s'étendant de manière distale, les saillies (30) sont comprimées vers l'espace intérieur.

2. Procédé pour préparer un dispositif de pose d'implant médical selon la revendication 1, pour le déploiement, composé des étapes consistant à :
positionner un implant médical sur un élément s'étendant de manière axiale du dispositif ;
faire passer l'unique butée de stent à ressort à travers le stent jusqu'à une position proximale du stent jusqu'à ce que le stent mette en prise par friction l'élément s'étendant de manière axiale ; et
fixer un composant de pointe distale sur l'extrémité la plus distale de l'élément s'étendant de manière axiale.
